# EUROPÄISCHE PATENTSCHRIFT

(11) **EP 2 036 510 B1**
(45) Veröffentlichungstag und Bekanntmachung des Hinweises auf die Patenterteilung: **06.10.2010**
(21) Anmeldenummer: 07450161.0
(22) Anmeldetag: 17.09.2007
(51) Int. Cl.: A61B 17/70

(54) **Vorrichtung für die Korrektur kyphotischer Deformationen der Wirbelsäule**
Device for correcting kyphotic deformations of the spine
Dispositif de correction de déformations cyphotiques de la colonne vertébrale

(43) Veröffentlichungstag der Anmeldung: 18.03.2009
(73) Patentinhaber: Laka, Aleksandr Andreewitsch, 109544 Moskau (RU); Tablichanowitsch, Sampiew Muchammad, 111531 Moskau (RU); Wasilewitsch, Sagorodni Nikolai, 123298 Moskau (RU)
(72) Erfinder: Laka, Aleksandr Andreewitsch, 109544 Moskau (RU); Tablichanowitsch, Sampiew Muchammad, 111531 Moskau (RU); Wasilewitsch, Sagorodni Nikolai, 123298 Moskau (RU)
(74) Vertreter: Haffner und Keschmann Patentanwälte OG

(56) Entgegenhaltungen:
- EP-A- 1 252 865
- WO-A-2007/045892

## Beschreibung

Die Erfindung betrifft eine Vorrichtung für die Korrektur kyphotischer Deformationen der Wirbelsäule bei Erwachsenen und Kindern, umfassend ein Paar biegsamer Platten für die Festlegung zu beiden Seiten der Dornfortsätze der Wirbel und eine Mehrzahl von Befestigungsmitteln zum Festlegen der Platten an den Wirbelbögen, wobei die Befestigungsmittel jeweils ein C-förmiges Glied zum Erfassen je eines Wirbelbogens und ein schraubenförmiges Element umfassen, das durch eine Bohrung in einem freien Ende eines Schenkels des C-förmigen Glieds und mit seinem Endabschnitt in den Wirbelbogen schraubbar ist und wobei ein Verbindungselement zur Verbindung des schraubenförmigen Elements mit der biegsamen Platte vorgesehen sind.

Eine solche Vorrichtung ist z.B. aus WO-A-942 11 86 bekannt.

Derartige Vorrichtungen können in der Traumatologie, der Orthopädie und der Vertebrologie zum Einsatz kommen.

Bekannt sind Vorrichtung der eingangs genannten Art für die Korrektur von Deformationen der Wirbelsäule, die ein Paar biegsamer Platten zur Befestigung entlang beider Seiten der Wirbelsäule aufweisen, und die mit Hilfe von über die Länge der Platten angeordneten Verbindungsblöcken an den Wirbeln festgelegt werden können. Bei den bekannten Vorrichtungen haben die Verbindungselemente jedoch nicht immer den hohen mechanischen Belastungen, wie Sie im Bereich der Wirbelsäule auftreten, standgehalten. Gelegentlich waren Brüche von Bauteilen zu beobachten, mit deren Hilfe die Platten jeweils klemmend festgelegt waren.

Die Erfindung zielt daher darauf ab, eine stabilere und bruchsichere Verbindung der Platten mit der Wirbelsäule zu schaffen, wobei gleichzeitig verhindert werden soll, dass Verbindungselemente das normalen Wachstum der Wirbelsäule in die Länge besonders bei Patienten von relativ jungem Alter behindern. Weiters zielt die Erfindung darauf ab, die Höhe der Konstruktion zu vermindern, um insbesondere bei der Implantation bei Kindern ein tieferes Absenken der Vorrichtung in weiches Gewebe zu ermöglichen, ohne dabei die korrigierende Kraft zu verlieren.

Zur Lösung dieser Aufgabe zeichnet sich die erfindungsgemäße Vorrichtung dadurch aus, dass das Verbindungselement eine Durchgangsbohrung für das schraubenförmige Element und eine außerhalb des Querschnitts der Durchgangsbohrung angeordnete, sich quer zur Durchgangsbohrung durch das Verbindungselement erstreckende Ausnehmung zur Aufnahme der Platte aufweist, wobei die Ausnehmung zu dem die Bohrung aufweisenden Schenkel des C-förmigen Glieds hin offen ist und der die Bohrung aufweisende Schenkel des C-förmigen Glieds einen seitlichen Ansatz aufweist, der eine Anlagefläche für die in der Ausnehmung aufgenommenen Platte ausbildet. Ein derartiges Verbindungselement stellt eine sichere Verbindung des schraubenförmigen Elements mit der Platte sicher, wobei dadurch, dass die sich quer zur Durchgangsbohrung durch das Verbindungselement erstreckende Ausnehmung zur Aufnahme der Platte außerhalb des Querschnitts der Durchgangsbohrung für das schraubenförmige Element angeordnet ist, sichergestellt ist, dass die Platte in der Ausnehmung festgespannt bzw. geklemmt werden kann, ohne dass die Stabilität der Festlegung durch seitliche Krafteinwirkung durch das in der Durchgangsbohrung aufgenommene schraubenförmige Element beeinträchtigt wird. Die Klemmung erfolgt lediglich dadurch, dass der dem Verbindungselement zugewandte Schenkel des C-förmigen Glieds einen seitlichen Ansatz aufweist, der eine Anlagefläche für die in der Ausnehmung aufgenommenen Platte ausbildet, sodass die Platte zwischen der Anlagefläche des seitlichen Ansatzes und der dazu parallelen Gegenfläche in der Ausnehmung des Verbindungselements verspannt werden kann. Im Vergleich zu Ausbildungen gemäß dem Stand der Technik werden für die Verklemmung der Platten keine zusätzlichen Elemente benötigt, die ggf. zwischen dem C-förmigen Glied und der Platte von dem schraubenförmigen Element getragen werden, und die durch eine außermittige Belastung einer besonders hohen Bruchgefahr ausgesetzt wären.

Die Verspannung kann hierbei gemäß einer bevorzugten Weiterbildung der erfindungsgemäßen Vorrichtung derart erfolgen, dass auf das aus dem Verbindungselement austauchende Ende des schraubenförmigen Elements eine Mutter aufgeschraubt ist, die gegen das Verbindungselement geschraubt ist.

Um eine entsprechende Klemmkraft aufbringen zu können, ist bevorzugt vorgesehen, dass die Platte eine Höhe aufweist, die größer ist als die Höhe der Ausnehmung des Verbindungselements.

Gleichzeitig mit der Verspannung der Platte kann durch das Aufschrauben der Mutter auf das aus dem Verbindungselement austauchende Ende des schraubenförmigen Elements auch eine Festlegung von Querträgern zur Verbindung der jeweils paarweise angeordneten biegsamen Platten erfolgen, die jeweils zu beiden Seiten der Dornfortsätze der Wirbel platziert werden. Zu diesem Zweck ist die erfindungsgemäße Ausbildung mit Vorteil derart weitergebildet, dass die schraubenförmigen Elemente paarweise miteinander durch Querträger verbunden sind, die von den schraubenförmigen Elementen durchsetzt sind und jeweils mit Hilfe der Mutter gegen die Verbindungselemente gespannt und festgelegt sind.

Um eine entsprechende Symmetrie innerhalb der jeweils paarweise angeordneten Verbindungselemente herzustellen und um den anatomischen Gegebenheiten der Wirbelsäule gerecht zu werden ist gemäß eine bevorzugten Weiterbildung der erfindungsgemäßen Vorrichtung vorgesehen, dass die seitlichen Ansätze der C-förmigen Glieder zweier ein Paar bildenden Befestigungselemente voneinander wegweisend angeordnet sind. Dies bedeutet mit Rücksicht auf den Umstand, dass die seitlichen Fortsätze jeweils eine Anlagefläche für die zu beiden Seiten der Wirbelsäule angeordneten Platten aufweisen, dass die Platten in Bezug auf die Wirbelsäule jeweils außerhalb der schraubenförmigen Elemente, das heißt in einem größeren seitlichen Abstand von der Wirbelsäulenmitte angeordnet sind als die schraubenförmigen Elemente. Dadurch kann die Stabilität verbessert und die von den biegsamen Platten ausgehende korrigierende Kraft auf die Wirbelsäule erhöht werden.

Um nun eine plane und sichere Anlage des seitlichen Ansatzes an der Platte zu begünstigen, ist die Ausbildung in vorteilhafter Weise derart getroffen, dass das schraubenförmige Element eine Verdickung aufweist, die einen Anschlag für den die Bohrung aufweisenden Schenkel des C-förmigen Glieds beim Einschrauben des schraubenförmigen Elements in den Wirbelbogen bildet, wobei in bevorzugter Weise die Durchgangsbohrung des Verbindungselements einen Abschnitt mit vergrößertem Durchmesser zum Aufnehmen der Verdickung aufweist. Bei einer derartigen Ausbildung kann das schraubenförmige Element bis zu der erwähnten Verdickung in das C-förmige Glied eingeschraubt werden, wobei durch Vorgabe des Anschlags reproduzierbare Verhältnisse und eine Begrenzung der Einschraubtiefe gegeben sind. Dadurch dass die Vertiefung nach Aufsetzen des Verbindungselements in einem Abschnitt der Durchgangsbohrung mit vergrößertem Durchmesser ausgenommen ist, kann die Klemmkraft voll und unmittelbar von dem seitlichen Ansatz auf die in der Ausnehmung aufgenommene Platte zur Wirkung gelangen.

Die Verdickung kann zudem eine zusätzliche Einschraubhilfe beim Einschrauben des schraubenförmigen Elements in das C-förmige Glied und insbesondere in den Wirbelbogen darstellen, zu welchem Zweck die Ausbildung bevorzugt derart weitergebildet ist, dass die Verdickung auf dem schraubenförmigen Element mit Kanten zum Angriff eines Werkzeugs für das Festschrauben des schraubenförmigen Elementes ausgebildet ist

Um eine Anpassung an unterschiedliche anatomische Ausbildungen der Wirbelsäule und insbesondere eine Verwendung der erfindungsgemäßen Korrekturvorrichtung auch bei Kindern zu ermöglichen, ist mit Vorteil vorgesehen, dass die C-förmigen Glieder in wenigstens zwei unterschiedlichen Größen verfügbar sind.

Zusammenfassend wird mit der vorliegenden Erfindung bevorzugt eine Vorrichtung für die Korrektur von Deformationen der Wirbelsäule geschaffen, die ein Paar biegsamer Platten zur Befestigung beiderseits der Wirbelsäule und eine Mehrzahl von entlang der Wirbelsäule angeordneten Befestigungsmitteln zum Festlegen der Platten an den Wirbelbögen aufweist, wobei die Befestigungsmittel C-förmige Glieder zweier Typengrößen für das Fassen der Wirbelbögen umfassen, die mit den in einen freien Schenkel eingeschraubten sich verjüngenden schraubenförmigen Elementen versehen sind, deren Größe an die Größe der C-förmigen Glieder angepasst ist. Von den C-förmigen Gliedern gibt es zwei Arten, die sich durch die Richtung des seitlichen Ansatzes, nach rechts oder nach links, als Stütze der biegsamen Platte links oder rechts vom Dornfortsatz, unterscheiden. Es ist jeweils ein Verbindungselement zum Verbinden der biegsamen Platte mit dem schraubenförmigen Element vorgesehen, welches eine erste Ausnehmung in Form einer Durchgangsbohrung für das Durchführen des schraubenförmigen Elementes und eine zweite Ausnehmung aufweist, welche die biegsame Platte erfasst. Für die endgültige Stabilisierung und Fixierung der Konstruktion werden Querträger verwendet, die auf die paarweise zusammengeführten schraubenförmigen Elemente und die Muttern aufgebracht werden, welche auf die schraubenförmigen Elemente bis zu dem Querträger aufzuschrauben sind und die den Block fixieren, wenn die Muttern angezogen sind.

Eine solche technische Lösung hat folgende Vorteile: sie sichert die Möglichkeit des gleichmäßigeren Wachstums der Wirbelsäule nach dem Anbringen dieser Vorrichtung an der Wirbelsäule. Sie weist einen kompakteren Aufbau auf und tritt nicht über das Gewebe heraus. Sie ermöglicht es, den Arbeitsaufwand und die Zeit der Operation zu verringern und ist weiters weniger traumatisch bei ihrem Einsatz bei der Operation.

Weiters ermöglicht es die Vorrichtung, dass bei der Durchführung einer Operation C-förmige Glieder der Größe ausgewählt werden, die der Größe des Wirbelbogens entsprechen. Die Vorrichtung ermöglicht es außerdem eine Beweglichkeit einzelner der schraubenförmigen Elemente mit den C-förmigen Gliedern relativ zu den biegsamen Platten in vorbestimmten Abschnitten der Platten zuzulassen, während in anderen Abschnitten eine derartige Beweglichkeit unterbunden werden kann. Je nachdem ob eine Beweglichkeit der Platten zuzulassen ist oder nicht, kann die Höhe der Ausnehmung des Verbindungselements, in welcher die Platte aufgenommen ist, geringer sein als die Höhe der biegsamen Platte oder größer als diese.

Bezüglich der Dimensionierung der Platten ist es weiters in bevorzugter Weise möglich, die Platten mit einer gegenüber dem Stand der Technik geringeren Höhe und einer größeren Breite auszuführen, wodurch ihre korrigierende Wirkung auf die Wirbelsäule verbessert wird.

Die Querträger können längs der biegsamen Platten auf unterschiedliche Weise angebracht werden. Zum Beispiel kann jedes Verbindungselement mit Querträgern ausgestattet werden. Aber möglich ist auch eine solche Variante, bei der die Verbindungselemente wahlweise mit Querträgern versehen werden.

Zur Erhöhung der Bequemlichkeit, zur Vereinfachung des Anbringens der Vorrichtung im Verlaufe der Operation und zur Verringerung der Zeit ist es von Vorteil, dass an jedem sich verjüngenden schraubenförmigen Element eine mutterförmige Verdickung vorhanden ist, von der aus nach der einen Seite der dem Verbindungselement zugewandte Schenkel des C-förmigen Gliedes und nach der anderen Seite das Verbindungselement angebracht ist. Dabei muss Letzteres eine Aussparung am Ende der Durchgangsbohrung aufweisen, die der Größe der mutterförmigen Verdickung entspricht, und in welche sie einzulassen ist.

Die sich verjüngenden schraubenförmigen Elemente können bevorzugt in zwei Größen verfügbar sein und an die entsprechenden Größen der C-förmigen Glieder angepasst sein.

Die Position der mutterförmigen Verdickung auf dem sich verjüngenden schraubenförmigen Element kann sich in solcher Entfernung vom spitzen Ende befinden, welche ein Eindringen des spitzen Endes des schraubenförmigen Elements in den Wirbelkanal beim Einschrauben ausschließt.

Dabei ist es zur Erhöhung der Bequemlichkeit des Festschraubens zweckmäßig, dass die mutterförmige Verdickung geformt ist, um als Werkzeug für das Festschrauben des schraubenförmigen Elementes zu dienen.

Die Erfindung wird nachfolgend anhand von in der Zeichnung dargestellten Ausführungsbeispielen näher erläutert. In dieser zeigen
Fig.1 eine Gesamtansicht der erfindungsgemäßen Vorrichtung für die Korrektur einer kyphotischen Deformation der Wirbelsäule,
Fig.2 eine Seitenansicht eines Ausschnitts der Fig.1,
Fig.3 einen Querschnitt entlang der Linie III-III der Fig.2,
Fig.4 eine Ansicht des in Fig.2 gezeigten Abschnitts von oben,
Fig.5 eine Detailansicht eines C-förmigen Glieds in einer ersten Größe,
Fig.6a und 6b eine Ansicht auf zwei Ausbildungen eines C-förmigen Glieds gemäß Fig.5 von oben,
Fig.7 eine Detailansicht eines C-förmigen Glieds in einer zweiten Größe,
Fig.8a und 8b eine Ansicht auf zwei Ausbildungen eines C-förmigen Glieds gemäß Fig.7 von oben,
Fig.9a und 9b einen Querschnitt eines Verbindungselements in einer ersten und in einer zweiten Größe,
Fig.10a und 10b Ansichten der Verbindungselemente gemäß den Fig.9a und 9b von oben,
Fig.11a und 11b Querschnitte entlang der Linie XI-XI der Fig.9a und 9b, und
Fig.12a und 12b eine Ansicht des schraubenförmigen Elements in zwei Größen.

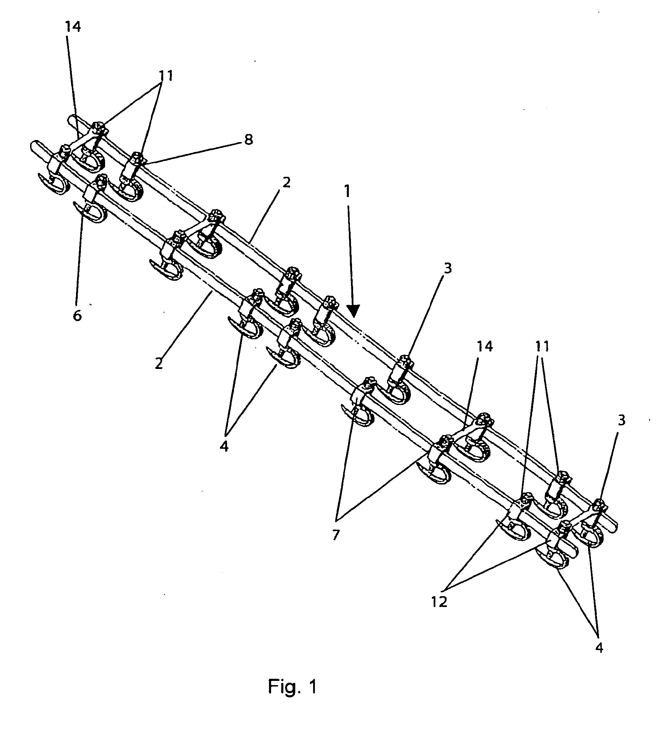

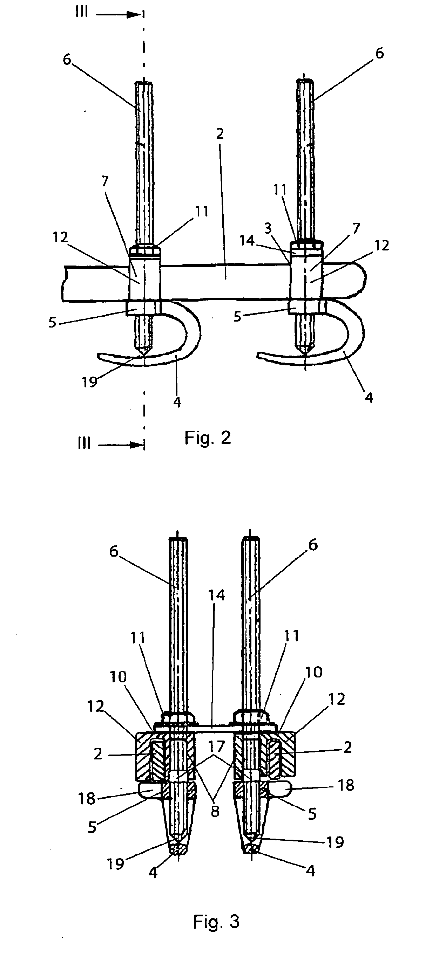

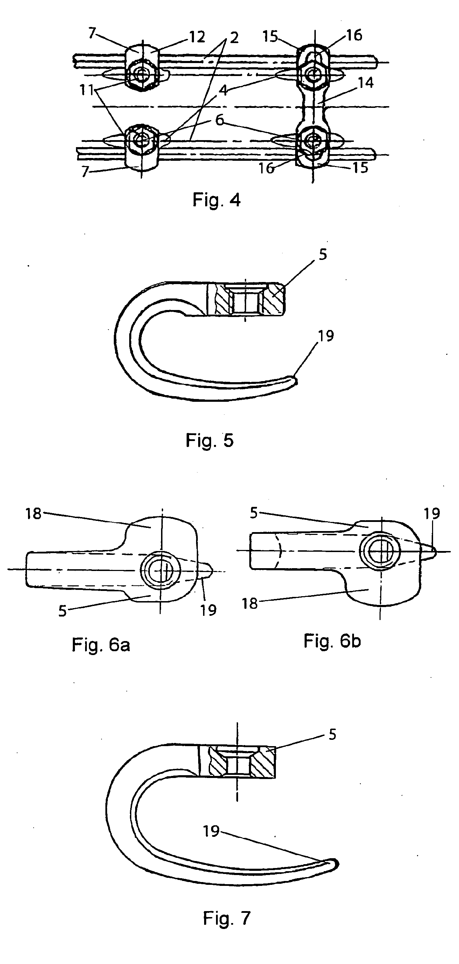

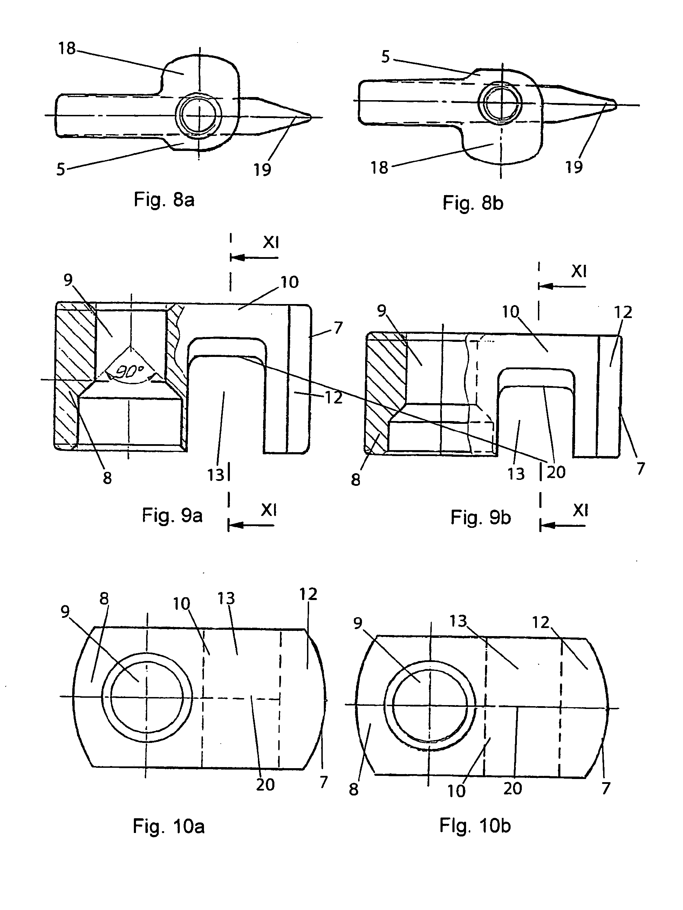

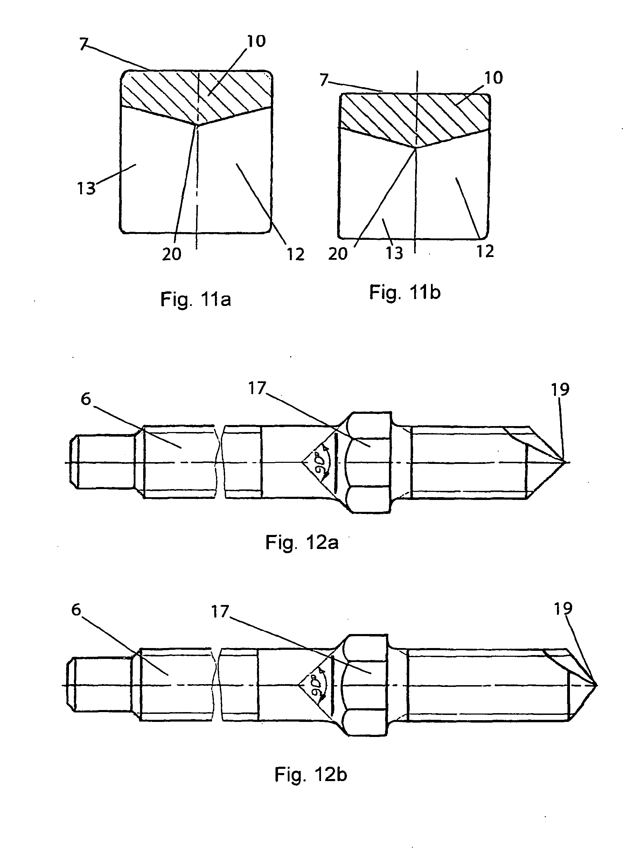

Die Vorrichtung 1 für die Korrektur von Deformationen der Wirbelsäule umfasst ein Paar biegsamer Platten 2 für das Anbringen beiderseits der Wirbelsäule, entlang derer Befestigungsmittel 3 zur Verbindung mit den Wirbeln gelegen sind. Jedes Befestigungsmittel 3 zur Verbindung mit den Wirbeln weist ein Paar C-förmiger Glieder 4 für das Fassen der Wirbelbögen mittels in deren Schenkel 5 eingeschraubter sich verjüngender schraubenförmiger Elemente 6, wobei die schraubenförmigen Elemente 6 mit Hilfe von Verbindungselementen 7 mit den biegsamen Platte 2 verbunden sind. Von den C-förmigen Gliedern 4 gibt es zwei Arten, und sie unterscheiden sich durch das Vorhandensein des seitlichen Ansatzes 18 rechts (Fig.6b und Fig.8b) oder links (Fig.6a und Fig.8a). Beim Einführen des C-förmigen Glieds 4 hinter den Wirbelbogen muss der seitliche Ansatz 18 nach rechts oder nach links vom Dornfortsatz gerichtet sein, so wie in Fig.3 dargestellt. Die C-förmigen Glieder haben zwei Größen - eine kleinere für das Fassen der Bögen im Oberbrust- und Brustbereich der Wirbelsäule (Fig.5) und eine größere für den Bereich der Lendenwirbelsäule (Fig.7). Für das kleinere oder größere C-förmigen Glied wird für die Fixierung am Wirbelbogen das schraubenförmige Element 6 entsprechender Größe verwendet (Fig.12). Dabei wird jedes schraubenförmige Element 6 mit Hilfe des Verbindungselements 7 (Fig.9 bis 11) mit der biegsamen Platte 2 verbunden. Die biegsame Platte 2 wird durch das Verbindungselements 7 über dem seitlichen Ansatz 18 des C-förmigen Glieds 4 fixiert. Außerdem ist die Vorrichtung mit Querträgern 14 versehen. Jeder der Blöcke 3 kann mit Querträgern 14 versehen sein oder es können wahlweise nur einige der Blöcke 3 mit Querträgern 14 versehen sein, so wie in Fig.1 gezeigt. Die Anzahl der Querträger 14 und die Stellen, an denen sie angebracht werden, werden vom Chirurgen im Verlaufe der Vorbereitung und Durchführung der Operation bestimmt.

Ebenfalls im Verlauf der Vorbereitung und Durchführung der Operation legt der Chirurg fest, an welchen Blöcken 3 die biegsamen Platten 2 fest mit den C-förmigen Gliedern 4 für das Fassen der Wirbelbögen verbunden werden müssen, und an welchen mit der Möglichkeit der Längsverschiebung entlang der Längsachse der biegsamen Platten. Für die Sicherstellung der oben genannten Längsverschiebung der biegsamen Platte 2 werden solche Verbindungselemente 7 ausgesucht, bei denen die Höhe des Ansatzes 8 mit der Durchgangsbohrung 9 die Höhe der biegsamen Platte 2 übersteigt, wobei die Letztere frei in der Ausnehmung 13 zwischen den Ansätzen 8 und 12 des Verbindungselements 7 angeordnet wird und eine geringere Höhe hat als die Höhe der Ausnehmung 13 (Fig.9a). In diesem Fall ist nach der Vorverdichtung der Überbrückung 10 durch die Mutter 11 zum Schenkel 5 des C-förmigen Glieds 4 die biegsame Platte 2 in der durch den seitlichen Ansatz 18 des C-förmigen Glieds 4 geschlossene Ausnehmung 13 des Verbindungselements 7 frei verschieblich angeordnet.

Für eine starre Verbindung mit der biegsamen Platte 2 werden solche Verbindungselemente 7 ausgewählt, bei denen die Höhe der Ausnehmung 13 geringer ist als die Höhe der biegsamen Platte 2 (Fig.9b). In diesem Fall ist nach der Vorverdichtung der Überbrückung 10 durch die Mutter 11 zum Schenkel 5 des C-förmigen Glieds 4 die biegsame Platte 2 unbeweglich in der Ausnehmung 13 des Verbindungselements 7 eingespannt, wobei die Platte 2 zwischen der Anlagefläche am seitlichen Ansatz 18 des C-förmigen Glieds 4 und der entsprechenden parallelen Gegenfläche in der Ausnehmung 13 festgeklemmt ist.

Die Querträger 14 können mit den biegsamen Platten 2 auf unterschiedliche Art und Weise verbunden werden. Die zweckmäßigste Konstruktion ist die, bei welcher das Endteil 15 des Querträger 14 zwischen der Überbrückung 10 des Verbindungselements 7 und der Mutter 11, welche das Verbindungselements 7 an den Schenkel 5 des C-förmigen Glieds 4 zum Fassen der Wirbelbögen drückt, geklemmt wird. Dabei kann der Querträger 14 eine Platte 2 mit durchgehender Bohrung 16 (Fig.4) an den Enden darstellen. Während des Befestigens der Vorrichtung mittels der Bohrungen 16 werden die Querträger 14 auf die freien Enden der schraubenförmigen Elemente 6 aufgesetzt. Nachdem die Überbrückungen mit einer Seite auf die Verbindungselemente 7 aufschlagen, wird damit begonnen, die Muttern 11 auf die schraubenförmigen Elemente 6 bis zu deren Verdichtung hin zur anderen Seite des Querträgers 14 festzuschrauben.

Auch die Konstruktion des sich verjüngenden schraubenförmigen Elementes 6 kann unterschiedlich sein. Zum Beispiel kann es als sich verjüngender zylindrischer Stab mit Schraubgewinde an der äußeren seitlichen Oberfläche ausgebildet sein. Jedoch ist eine Konstruktion am zweckmäßigsten, bei welcher an jedem sich verjüngenden schraubenförmigen Element 6 eine mutterförmige Verdickung 17 vorhanden ist, von der in Richtung der einen Seite der Schenkel 5 des C-förmigen Glieds 4 für das Fassen der Wirbelbögen gelegen ist, und in Richtung der anderen Seite die mutterförmige Verdickung 17 in die Aussparung der Durchgangsbohrung 9 des Verbindungselements 7 (Fig.12) eingelassen wird.

Zur Erhöhung der Bequemlichkeit beim Festschrauben jedes sich verjüngenden Elementes 6 in den Wirbelbogen ist es wünschenswert, dass die mutterförmige Verdickung 17 in der Art eines Werkzeuges für das Festschrauben des sich verjüngenden schraubenförmigen Elementes 6 ausgeführt ist.

In der Folge wird der typische Ablauf einer Operation unter Verwendung der erfindungsgemäßen Vorrichtung dargestellt. Durch linearen Schnitt über den Dornfortsätzen vom ersten Brustwirbel bis zum ersten Kreuzwirbel werden die Wirbelbögen schichtweise freigelegt und durch Ablösen der Muskeln vom Knochen mit Hilfe eines Schabers skelettiert. Danach werden nacheinander die C-förmige Glieder 4 unter den Wirbelbogen geführt, und die sich verjüngenden schraubenförmigen Elemente 6 werden in die mit einem Innengewinde versehene Bohrung im Schenkel 5 eingeschraubt. Vor dem Einführen des C-förmigen Glieds 4 wird die Dicke des Wirbelbogens ermittelt, und das C-förmige Glied 4 wird entsprechend seiner Größe ausgewählt. Die Einführung des C-förmigen Glieds 4 hinter den Wirbelbogen wird an der Basis des Dornfortsatzes des Wirbels so durchgeführt, dass der seitliche Ansatz 18 des C-förmigen Glieds 4 vom Dornfortsatz aus zur entgegengesetzten Seite gerichtet ist. Im Verlaufe des Einschraubens durchdringt das sich verjüngende Ende 19 des schraubenförmigen Elementes 6 den Wirbelbogen. Das Einschrauben wird bis zum Anschlag der Schenkels 5 des C-förmigen Glieds 4 fortgesetzt. Das Anhalten des Einschraubens im Ergebnis des genannten Anschlags erfolgt in dem Moment, wenn sich zwischen dem sich verjüngenden Ende 19 des schraubenförmigen Elementes 6 und der inneren Oberfläche des C-förmigen Glieds 4 ein unbedeutender Abstand befindet. Im Ergebnis ist der Wirbelbogen fest mit dem C-förmigen Glied 4 verbunden. Danach wird zu dem Wirbelbogen übergegangen, der gegenüber, in Richtung der anderen Seite vom Dornfortsatz aus gelegen ist, und analog wird an ihm das C-förmige Glied 4 angebracht. Im Ergebnis bildet sich ein Block 3 der Verbindung mit den Wirbeln.

Weiterhin werden auf analoge Art und Weise die folgenden Blöcke 3 der Verbindung mit den Wirbeln geschaffen, oder anders gesagt, es wird die erforderliche Anzahl an Paaren der C-förmigen Gliedern 4 mit den in sie eingeschraubten sich verjüngenden schraubenförmigen Elementen 6 längs der Wirbelsäule geschaffen, wobei alle C-förmigen Glieder 4 an den Wirbelbögen fixiert werden. Die Anzahl an Paaren C-förmiger Glieder 4 (oder Blöcken 3 der Verbindung mit den Wirbeln) kann unterschiedlich sein. Zum Beispiel von neun bis zwölf oder mehr. Ihre Anzahl hängt vom Grad der Verkrümmung der Wirbelsäule und deren Form ab.

Hierauf werden die Art der Deformation der Wirbelsäule und die Richtung der Verkrümmung bestimmt. Unter Berücksichtigung dessen werden die biegsamen Platten 2 genommen und sie werden in zwei zueinander senkrechten Ebenen und in die Richtungen gebogen, die den Deformationen der Wirbelsäule entgegengesetzt sind, mit dem Ziel, dass es nach Festlegung der Vorrichtung an der Wirbelsäule zu einer Aufrichtung der Wirbelsäule kommt. Deshalb wird jede biegsame Platte 2 in der Sagittalebene unter Berücksichtigung der Brustbiegung und der Lordose gebogen, wobei die physiologischen Biegungen der Wirbelsäule wiederholt werden. Danach in der Frontalebene in der Richtung, die der Deformation der Wirbelsäule entgegengesetzt ist, für deren nachfolgende Korrektur. Gleichzeitig hiermit wird vor dem Anbringen der biegsamen Platten 2 in Abhängigkeit von der Art der Deformation festgelegt, welche sich verjüngenden schraubenförmigen Elemente 6 mit den C-förmigen Gliedern 4 fest an der entsprechenden biegsamen Platte 2 befestigt werden müssen, und welche mit der Möglichkeit der Längsverschiebung nach dem Anziehen der Muttern 11. Dabei kann in einem Block 3 der Verbindung mit den Wirbeln sowohl eine feste Verbindung mit der biegsamen Platte 2 als auch solche mit der Möglichkeit der Längsverschiebung der biegsamen Platte 2 vorhanden sein.

Danach wird das Anbringen der biegsamen Platten 2 vorgenommen. Dazu wird zuerst eine biegsame Platte 2 genommen, und man drückt sie mit der Seite von der einen Seite aller schraubenförmigen Elemente 6 von einer Seite der Wirbelsäule an. Nach dem Andrücken wird mit zuerst ein Verbindungselement 7 auf ein sich verjüngendes schraubenförmiges Element 6 so aufgereiht, dass das schraubenförmige Element 6 die Durchgangsbohrung 9 durchsetzt und das Verbindungselement mit dem Ansatz 12 die biegsame Platte 2 erfasst, welche sich dann innerhalb der Ausnehmung 13 befindet. Danach wird das zweite Verbindungselement 7 genommen und in analoger Weise auf das zweite sich verjüngende schraubenförmige Element 6 aufgereiht. Auf diese Art und Weise erfolgt nacheinander das Aufsetzen der Verbindungselemente 7 auf alle sich verjüngenden schraubenförmigen Elemente 6 an der einen Seite der Wirbelsäule sowie die Fixierung der biegsamen Platte 2 an diesen.

Daraufhin wird dasselbe an der anderen Seite der Wirbelsäule getan. Im Ergebnis sind beiderseits der Wirbelsäule die biegsamen Platten 2 befestigt, die mittels der sich verjüngenden schraubenförmigen Elemente 6 mit den C-förmigen Gliedern 4 verbunden sind, welche ihrerseits mit den Wirbelbögen verbunden sind.

Danach werden die Enden der sich verjüngenden schraubenförmigen Elemente 6 eines Blocks 3 der Verbindung mit den Wirbeln untereinander zusammengeführt. der Querträger 14 wird aufgelegt, indem man über dessen durchgehende Bohrungen 16 die genannten Enden der sich verjüngenden schraubenförmigen Elemente 6 eines Blocks 3 der Verbindung mit den Wirbeln durchzieht. Im Ergebnis wird der Querträger durch die Endteile von oben auf die Überbrückung 10 der Verbindungselemente 7 gelegt. Daraufhin wird auf jedes sich verjüngende schraubenförmige Element 6 eine Mutter 11 bis zum Anschlag an die obere Oberfläche des Querträgers 14 aufgeschraubt und angezogen.

Nun wird zu einem anderen Paar von sich verjüngenden schraubenförmigen Elementen 6 des nächsten Blocks 3 der Verbindung mit den Wirbeln übergegangen und es werden die analogen Handlungen ausgeführt. Dabei werden, falls diese Notwendigkeit besteht, Querträger 14 in der im vorigen Absatz genannten Weise angebracht. Besteht eine solche Notwendigkeit nicht, beschränkt man sich auf das Aufschrauben der Muttern 11 auf die sich verjüngenden schraubenförmigen Elemente 6 bis zum Anschlag an die obere Oberfläche des Verbindungselements 7, worauf die Mutter 11 festgezogen wird.

Nach dem Abschluss des Festschraubens der Muttern erfolgt das "Abknipsen" der Teile der sich verjüngenden schraubenförmigen Elemente 6 der Blöcke 3 der Verbindung mit den Wirbeln gleich über den Muttern 11, damit sie nicht über die Konstruktion hinausragen.

Zwischen dem Verbindungselement 7 und der biegsamen Platte 2 kann auf der Überbrückung innerhalb der Ausnehmung 13 ein vorstehender Steg 20 angeordnet werden.

Das Wesen der Erfindung wird nachfolgend an Hand eines klinischen Beispiels erläutert.

Patientin P., 9 Jahre alt, kam mit der Diagnose idiopathische rechtsseitige C-förmige Seitwärtsverkrümmung der Wirbelsäule IV. Grades. Auf dem Röntgenbild wurde eine rechtsseitige Brustskoliose von 96° nach Cobb festgestellt. Nach den Worten der Patientin wurde die Deformation erstmals im Alter von 8 Jahren entdeckt. Die konservative Therapie brachte im Verlauf von einem Jahr keine positive Dynamik. Der Patientin wurde eine operative Therapie vorgeschlagen. Die Operation wurde durchgeführt: Korrektur der seitlichen Deformation der Wirbelsäule durch dorsalen Eingriff in einem Schritt. Während der Operation wurde in einem Schritt die Korrektur mit anschließender Fixierung der Wirbelsäule mittels der vorgeschlagenen Vorrichtung LSZ-4 vom ersten Brustwirbel bis zum fünften Lendenwirbel der Wirbelsäule vorgenommen. Die Deformation verringerte sich von 96° auf 15° nach Cobb, was 84% entspricht. Die Dauer der Operation betrug 1 Stunde und 40 Minuten, während sie bei Verwendung einer Vorrichtung gemäß dem Stand der Technik mehr als zwei Stunden gedauert hätte. Die Verkürzung der Zeit konnte dank der Nutzung der prinzipiell neuen Methode der Korrektur mit Hilfe biegsamer Platten, der neuen Technologie der Befestigung der biegsamen Platten an den Wirbelbögen, bequemerer Instrumente und der verringerten Anzahl von Einzelteilen der Konstruktion erreicht werden. Der operative Eingriff, der die Befestigung der Konstruktion einschließt, und die eigentliche Korrektur der Deformation in einem Schritt zur Folge hat, unterscheidet sich von früheren Möglichkeiten durch weniger verletztes Gewebe (Rückenmuskeln) und weniger Blutverlust (er beträgt im Durchschnitt 500 ml). Im Zeitraum nach der Operation befand sich die Patientin in einem normalen Bett. Nach dem Aufwachen aus der Narkose konnte die Patientin sowohl auf Rücken, Bauch oder Seite liegen. Am zweiten Tag war sie in der Lage, mit kleinen Übungen im Bett zu beginnen. Es wurde bei ihr eine Röntgenaufnahme gemacht und das Ergebnis der Operation bewertet. Die Anwendung der röntgenologischen Kontrolle während der Operation ist bei dieser Methode nicht erforderlich. Die Röntgenaufnahme zeigte, dass die Position der Vorrichtung genau den Erfordernissen entspricht. Die Restdeformation betrug 15°. Die Patientin klagte lediglich über Schmerzen im Bereich der Operationswunde, die mit Analgetika behandelt wurden. Am dritten Tag wurde die Patientin aktiviert (es wurde ihr gestattet aufzustehen und zu gehen). Mittel für eine äußere Fixierung (Korsett) wurden nicht eingesetzt. Die Intensität der Schmerzen im Bereich der Operationswunde ließ nach. Am zehnten Tag wurden die Nähte entfernt. Es gab keine Klagen über Schmerzen im Bereich der Operationswunde. Die Patientin wurde zur ambulanten Rehabilitationsbehandlung am Wohnort entlassen. Nach zwei Monaten wurde während einer ambulanten Kontrolluntersuchung eine Röntgenuntersuchung der Wirbelsäule vorgenommen. Die Lage der Vorrichtung war richtig. Es bestanden keine Korrekturverluste. Schmerzen plagten die Patientin nicht. Die Patientin war zu ihrer gewohnten Lebensweise zurückgekehrt. Das Ergebnis der Behandlung war gut.

Bei der Verwendung anderer Vorrichtungen ist die Durchführung einer so schnellen Operation mit wesentlichem Effekt nicht möglich. Die Operation unter Nutzung der angemeldeten Vorrichtung ist für den Patienten weniger traumatisch und für den Chirurgen weniger arbeitsintensiv. Außerdem besteht ein Vorteil der Operation unter Nutzung der erfindungsgemäßen Vorrichtung darin, dass im fernen postoperativen Zeitraum die Möglichkeit der Verlagerung der Fixierungsblöcke entlang der biegsamen Platte je nach Wachstum der Wirbelsäule gegeben ist. Die Anwendung der Vorrichtung ist bei Kindern, die das Wachstum nicht abgeschlossen haben, in frühen Stadien der Erkrankung möglich. Für die stabile unbewegliche Fixierung der biegsamen Platten kann jede beliebige Position an der Wirbelsäule auf Entscheidung des Chirurgen ausgewählt werden.

Auf diese Weise ermöglicht es die Erfindung, Deformationen der Wirbelsäule in drei Ebenen bedeutend zu verringern, die Rehabilitationszeit wesentlich zu verkürzen und damit die Gesamtdauer der Arbeitsunfähigkeit zu reduzieren.

## Patentansprüche

1. Vorrichtung für die Korrektur kyphotischer Deformationen der Wirbelsäule bei Erwachsenen und Kindern, umfassend ein Paar biegsamer Platten (2) für die Festlegung zu beiden Seiten der Dornfortsätze der Wirbel und eine Mehrzahl von Befestigungsmitteln (3) zum Festlegen der Platten (2) an den Wirbelbögen, wobei die Befestigungsmittel (3) jeweils ein C-förmiges Glied (4) zum Erfassen je eines Wirbelbogens und ein schraubenförmiges Element (6) umfassen, das durch eine Bohrung in einem freien Ende eines Schenkels des C-förmigen Glieds (4) und mit seinem Endabschnitt in den Wirbelbogen schraubbar ist und wobei ein Verbindungselement (7) zur Verbindung des schraubenförmigen Elements (6) mit der biegsamen Platte (2) vorgesehen sind, **dadurch gekennzeichnet, dass** das Verbindungselement (7) eine Durchgangsbohrung für das schraubenförmige Element (6) und eine außerhalb des Querschnitts der Durchgangsbohrung angeordnete, sich quer zur Durchgangsbohrung durch das Verbindungselement (7) erstreckende Ausnehmung (13) zur Aufnahme der Platte aufweist, wobei die Ausnehmung zu dem die Bohrung aufweisenden Schenkel des C-förmigen Glieds (4) hin offen ist und der die Bohrung aufweisende Schenkel des C-förmigen Glieds (4) einen seitlichen Ansatz (18) aufweist, der eine Ablagefläche für die in der Ausnehmung aufgenommenen Platte (2) ausbildet.

2. Vorrichtung nach Anspruch 1, **dadurch gekennzeichnet, dass** auf das aus dem Verbindungselement (7) austauchende Ende des schraubenförmigen Elements (6) eine Mutter aufgeschraubt ist, die gegen das Verbindungselement (7) geschraubt ist.

3. Vorrichtung nach Anspruch 1 oder 2, **dadurch gekennzeichnet, dass** die schraubenförmigen Elemente (6) paarweise miteinander durch Querträger verbunden sind, die von den schraubenförmigen Elementen (6) durchsetzt sind und jeweils mit Hilfe der Mutter gegen die Verbindungselemente (7) gespannt und festgelegt sind.

4. Vorrichtung nach Anspruch 1, 2 oder 3, **dadurch gekennzeichnet, dass** die seitlichen Ansätze der C-förmigen Glieder (4) zweier ein Paar bildenden Befestigungselemente voneinander wegweisend angeordnet sind.

5. Vorrichtung nach einem der Ansprüche 1 bis 4, **dadurch gekennzeichnet, dass** das schraubenförmige Element (6) eine Verdickung aufweist, die einen Anschlag für den die Bohrung aufweisenden Schenkel des C-förmigen Glieds (4) beim Einschrauben des schraubenförmigen Elements (6) in den Wirbelbogen bildet.

6. Vorrichtung nach einem der Ansprüche 1 bis 5, **dadurch gekennzeichnet, dass** die Durchgangsbohrung des Verbindungselements (7) einen Abschnitt mit vergrößertem Durchmesser zum Aufnehmen der Verdickung aufweist.

7. Vorrichtung nach einem der Ansprüche 1 bis 6, **dadurch gekennzeichnet, dass** die Platte (2) eine Höhe aufweist, die größer ist als die Höhe der Ausnehmung des Verbindungselements (7).

8. Vorrichtung nach einem der Ansprüche 1 bis 7, **dadurch gekennzeichnet, dass** die C-förmigen Glieder (4) in wenigstens zwei unterschiedlichen Größen verfügbar sind.

9. Vorrichtung nach einem der Ansprüche 1 bis 8, **dadurch gekennzeichnet, dass** die Verdickung auf dem schraubenförmigen Element (6) mit Kanten zum Angriff eines Werkzeugs für das Festschrauben des schraubenförmigen Elementes (6) ausgebildet ist.

## Claims

1. Device for the correction of kyphotic deformations of the spinal column in adults and infants, comprising a pair of flexible plates (2) for the fixation on both sides of the dorsal process of the vertebrae and a plurality of fixation means (3) for fixing the plates (2) to the vertebral arches, whereby the fixation means (3) each comprises a c-shaped member (4) for gripping of a vertebral arch each and a helical element (6), that is screwable through a bore in a free end of an arm of the c-shaped member (4) and with its end portion into the vertebral arch and whereby a connecting element (7) for connecting the helical element (6) with the flexible plate (2) is provided, **characterised in that** the connecting element (7) features a through-hole for the helical element (6) and a recess (13), arranged outside of the profile of the through-hole, running transversely to the through-hole through the connecting element (7) for the reception of the plate, whereby the recess is open towards the arm of the c-shaped member (4) having the bore and the arm of the c-shaped member (4) having the bore has a lateral lip (18) that forms a contact surface for the plate (2) received in the recess.

2. Device according to claim 1, **characterised in that** a nut is screwed onto the end of the helical element (6) emerging from the connecting element (7), which is screwed against the connecting element (7).

3. Device according to claim 1 or 2, **characterised in that** the helical elements (6) are connected with each other in pairs by cross bars, which are penetrated by the helical elements (6) and are each braced and fixed against the connecting elements (7) by the aid of the nut.

4. Device according to claim 1, 2 or 3, **characterised in that** the lateral lips of the c-shaped members (4) of two connecting elements forming a pair are arranged pointing away from each other.

5. Device according to any one of claims 1 to 4, **characterised in that** the helical element (6) bears a bulge that forms a stop for the arm of the c-shaped member (4) bearing the bore during screwing in of the helical element (6) in the vertebral arch.

6. Device according to any one of claims 1 to 5, **characterised in that** the through-hole of the connecting element (7) features a section with enlarged cross-section for the accommodation of the bulge.

7. Device according to any one of claims 1 to 6, **characterised in that** the plate (2) has a height, which is bigger than the height of the recess of the connecting element (7).

8. Device according to any one of claims 1 to 7, **characterised in that** the c-shaped members (4) are available in at least two sizes.

9. Device according to any one of claims 1 to 8, **characterised in that** the bulge on the helical element (6) has edges for gripping with a tool for the tightening of the helical element (6).

## Revendications

1. Dispositif pour corriger des déformations cyphotiques de la colonne vertébrale chez des adultes et des enfants, comportant une paire de plaques (2) flexibles, destinées à être fixées sur les deux côtés des apophyses épineuses des vertèbres, et une pluralité de moyens de fixation (3), destinés à fixer les plaques (2) contre les arcs neuraux, lesdits moyens de fixation (3) comportant chacun un élément (4) en forme de C, destiné à saisir respectivement un arc neural, et un élément (6) en forme de vis, qui peut être vissé à travers une forure disposée dans une extrémité libre d'une branche de l'élément (4) en forme de C et avec sa zone d'extrémité dans l'arc neural, et un élément d'assemblage (7) étant prévu pour assembler l'élément (6) en forme de vis à la plaque (2) flexible, **caractérisé en ce que** l'élément d'assemblage (7) comporte une forure débouchante pour l'élément (6) en forme de vis et un évidement (13) destiné à recevoir la plaque, lequel est disposé en dehors de la section de la forure débouchante et s'étend transversalement à la forure débouchante à travers l'élément d'assemblage (7), ledit évidement étant ouvert vers la branche, munie de la forure, de l'élément (4) en forme de C, et la branche, munie de la forure, de l'élément (4) en forme de C comportant une saillie latérale (18) qui forme une surface d'appui pour la plaque (2) logée dans l'évidement.

2. Dispositif selon la revendication 1, **caractérisé en ce que** sur l'extrémité de l'élément (6) en forme de vis, laquelle s'avance hors de l'élément d'assemblage (7), est posé un écrou qui est vissé contre l'élément d'assemblage (7).

3. Dispositif selon la revendication 1 ou 2, **caractérisé en ce que** les éléments (6) en forme de vis sont reliés par paires entre eux au moyen de traverses, à travers lesquelles passent les éléments (6) en forme de vis, et sont serrés et fixés respectivement au moyen de l'écrou contre les éléments d'assemblage (7).

4. Dispositif selon la revendication 1, 2 ou 3, **caractérisé en ce que** les saillies latérales des éléments (4) en forme de C de deux éléments de fixation formant une paire sont disposées en s'écartant l'une de l'autre.

5. Dispositif selon l'une quelconque des revendications 1 à 4, **caractérisé en ce que** l'élément (6) en forme de vis comporte un renflement qui, au moment du vissage de l'élément (6) en forme de vis dans l'arc neural, forme une butée pour la branche, munie de la forure, de l'élément (4) en forme de C.

6. Dispositif selon l'une quelconque des revendications 1 à 5, **caractérisé en ce que** la forure débouchante de l'élément d'assemblage (7) comporte un tronçon avec un plus grand diamètre, destiné à recevoir le renflement.

7. Dispositif selon l'une quelconque des revendications 1 à 6, **caractérisé en ce que** la plaque (2) a une hauteur qui est supérieure à la hauteur de l'évidement de l'élément d'assemblage (7).

8. Dispositif selon l'une quelconque des revendications 1 à 7, **caractérisé en ce que** les éléments (4) en forme de C sont disponibles au moins dans deux tailles différentes.

9. Dispositif selon l'une quelconque des revendications 1 à 8, **caractérisé en ce que** le renflement sur l'élément (6) en forme de vis est réalisé avec des bords pour la mise en application d'un outil utilisé pour serrer par vissage l'élément (6) en forme de vis.
